# EUROPEAN PATENT SPECIFICATION

(11) **EP 2 481 442 B1**
(45) Date of publication and mention of the grant of the patent: **22.01.2020**
(21) Application number: 09849652.4
(22) Date of filing: 22.09.2009
(51) Int. Cl.: A61M 27/00, A61M 1/00, A61F 13/02

(54) **VACUUM SEALING DRAINAGE DEVICE FOR HEALING WOUND ON BODY SURFACE**
VAKUUMVERSIEGELNDE DRAINAGEVORRICHTUNG ZUR WUNDHEILUNG AUF EINER KÖRPERFLÄCHE
DISPOSITIF DE DRAINAGE À ÉTANCHÉITÉ À VIDE POUR SOIGNER UNE BLESSURE SUR LA SURFACE DU CORPS

(43) Date of publication of application: 01.08.2012
(73) Proprietor: Wuhan VSD Medical Science & Technology Co., Ltd., Wuhan, Hubei 430022 (CN)
(72) Inventor: SONG, Jiuhong, Wuhan Hubei 430022 (CN)
(74) Representative: Zeuner Summerer Stütz
(86) International application number: PCT/CN2009/074088
(87) International publication number: WO 2011/035464

(56) References cited:
- CN-A- 1 571 682
- CN-A- 101 390 790
- CN-A- 101 390 790
- CN-A- 101 474 432
- DE-A1-102005 015 878
- US-A- 6 071 267
- US-A1- 2002 150 720
- US-A1- 2003 040 687
- US-A1- 2007 032 755
- US-A1- 2007 225 663
- US-A1- 2007 225 663
- US-A1- 2008 208 171

## Description

### FIELD OF THE INVENTION

The invention relates to a vacuum sealing drainage device for a bleeding wound tissue, and more particularly to a treating device that is designed for treatment of injury areas of various body surface tissues and performs vacuum sealing drainage treatment on a wound surface caused by injury, burn, infection, compression, endocrine dyscrasia, vascular occlusion, radioisotope exposure, surgeries, surgical complications, insect and snake bite, cryogenic burns, and so on.

### BACKGROUND OF THE INVENTION

A vacuum sealing drainage technology (VSD for short) is invented by Dr. Wim Fleischman from ULM University of Germany in 1992 and theory thereof has been formed, and it is for drainage of wound surface of limbs. In 1994, Chinese professor Qiu Huade first applied the VSD technology to general surgical departments, and initiated application of the VSD technology in the general surgical departments. Professors Wang Yanfeng and Qiu Huade got the first patent of the VSD technology with a patent number CN 2276350 on March 18, 1998 .

A basic configuration of the conventional VSD technology comprises a vacuum source (comprising a medical suction unit, a central vacuum unit, or a vacuum drainage bottle used in a hospital), a drainage piping, polyvinyl alcohol foams or other medical multi-hole foams, sponges or gauzes (a porous foam pad for short), a breathable film for adhesion and sealing (a sealing film for short), a connector (comprising a two-way connector, a three-way connector, or multi-way connector), and a drainage container. A structure thereof is as follow. A drainage piping with a side hole on one end thereof (normally two pipes parallel to each other, one pipe for a small porous foam pad, and three or more pipes parallel to each other for a comparatively big one) is inserted into the porous foam pad with a hole. As multiple porous foam pads are used, outlets of the drainage piping are combined into one outlet via the three-way connector, the two-way connector, or the multi-way connector. Then the vacuum source is connected for drainage, whereby conducting liquefaction materials of body tissues such as exudation, cataclysm, liquefaction necrosis tissue fragments, pus and so on into the drainage container.

In use, the drainage piping with multiple side holes is wrapped with the porous foam pad, and disposed on the wound surface or in a wound cavity. The sealing film is used to tightly seal the porous foam pad and an outlet of the drainage piping whereby separating them from the outside. Then the drainage container is connected, and finally the vacuum source is connected (or a vacuum drainage bottom made by combing the drainage container with the vacuum source), and thus a high-efficient drainage system (VSD system) is formed. In this system, negative pressure is transferred to the porous foam pad via rigid transmission of the drainage piping, and is distributed on every point on the porous foam pad along a trend of the drainage piping. Since vesicles in the porous foam pad are connected to each other and are rich in flexibility, negative pressure can reach every point of a targeted drainage area whereby forming omnibearing drainage. Under the action of the negative pressure, comparatively big, tender and blocky educts are cut and molded into granules, which enter the drainage piping via openings of the porous foam pad or vesicles connected to each other, and then are quickly inhaled into the drainage container. Big educts that may block the drainage piping are stopped by the porous foam pad, adhered to the surface of the porous foam pad, and left a body of the device along with the porous foam pad as drain is removed or replaced. Closing of the sealing film maintains negative pressure operating as drainage force, and separates the drainage area from the outside, which effectively prevent pollution and cross infection. Since negative pressure is uniformly distributed on the surface of the drainage area via the soft porous foam pad operating as a medium, it is capable of effectively preventing complications, such as ischemia, necrosis, perforation and so on, caused by suction of organs or tissues as one drainage piping is used for vacuum drainage.(referring to Page 3 to Page 4 of the second version of VSD technology edited by Qiu Huade and Song Jiuhong and published by People Health Publishing House)

Compared with normal dressing change, the VSD technology is capable of timely removing exudates and necrotic tissues in the drainage area, and thus the drainage area can enter a "zero accumulation" state such that the wound surface can quickly obtains a clean environment, and heavy absorption of toxins by the body can be greatly reduced. Even if a comparatively big cavity gap exists, the cavity gap is quickly reduced due to an existence of the negative pressure. For superficial wound surface, the sealing film and the porous foam pad cause a local environment to be more close to a physiological humid state. Negative pressure stimulation on soft tissues of the wound surface is helpful for improvement of a local microenvironment and subsidence of a tissue edema, accelerates regeneration of granulation tissues in the wound surface, and decreases healing time of the wound surface. No dressing change is required within 3-7 days of VSD treatment, which reduces workload of medics, alleviates pain of patients, and reduces overall medical cost.

In China, the VSD technology is widely applied to fields such as traumatology departments, orthopedics departments, general surgery departments, burn departments, and so on.

However, during use of the conventional VSD technology in drainage of a bleeding wound tissue, several problems exist: 1) sealing thereof is inconvenient. Good sealing is a key for ensuring drainage effect and the most difficult step during the whole vacuum sealing drainage. Common-used sealing methods include: a method of stabbing holes, a mesentery method, a suture method, a dumpling method, and so on (referring to Page 3 to Page 4 of the second version of VSD technology edited by Qiu Huade and Song Jiuhong and published by People Health Publishing House). However, the above-mentioned methods are very complex during practical operation, and operative doctors can grasp them after repeated operations and practices. In addition, infirm sealing often causes air leakage, and further decreasing of treatment effect or even treatment failure of the VSD system. 2) a leading-out end of the drainage piping is used as an outlet for negative-pressure conduction and drainage. There are few directions of the outlet for the negative-pressure conduction and the drainage, and the negative-pressure conduction and the drainage often occur in a single horizontal or a vertical direction, which easily cause non-uniform negative pressure conduction and pipe blockage. Moreover, as pipe blockage occurs, washing is inconvenient. Conventional washing methods include: 1. setting another rubber pipe on the wound surface, and using a mesentery method to seal a leading-out position of the rubber pipe, which increases possibility of "air leakage" and difficulty of sealing; 2. inversely injecting washing flushing liquid from an original drainage piping, which may re-inject drainage exudates on the wound surface and cause "reverse infection"; 3. stabbing a hole on and injecting flushing liquid into the porous foam pad, and the sealing film needs to be adhered to the hole on the porous foam pad, which increases operation difficulty. 3) it is inconvenient for drainage of massive and irregular wound surface. Leading-out ends of the drainage piping are connected via connectors, and a large amount of connectors are used to combine outlet ends of the drainage piping into one outlet. And thus an intersected mesh structure like a branch is formed, which makes movement of a patient very inconvenient. 4) in clinical use, the sealing film is used to seal the porous foam pad with healthy skin, since follicles in the healthy skin need normal permeable and breathable metabolism, permeability of the sealing film should be as large as possible, otherwise phenomenon such as skin rash, folliculitis, blushing caused by water immersion and so on may occur on the healthy skin, which causes a patient to consciously or unconsciously scratch and rub the sealing film due to troublesome symptoms such as itching and so on, and also causes stickiness of the sealing film to disappear and to detach from the skin surface due to water immerses between the skin and the sealing film, and in turn, causes air to enter a sealing area resulting in decreasing of effect or complete failure of vacuum sealing drainage. The porous foam pad requires complete sealing and permeability of the sealing film to be as small as possible, otherwise water loss will occur on the porous foam pad due to long-term and small airflow that causes the porous foam pad to become dry, and finally the porous foam pad loses elasticity and a humid environment, and healing time of the wound surface is increased. Thus a contradiction between two opposite reaction principles is formed. The conventional sealing film cannot simultaneously meet requirements for normally permeable, breathable, and metabolism skin, and for firm sealing of the porous foam pad. 5) for a massive wound surface, as two or more porous foam pads are needed, normally a drainage piping with a side hole in the cushion is pulled out, and after that an operative doctor cuts more side holes on the drainage piping, extends a length of the drainage piping with the side holes, and joints, serially connects, or parallel connects it with the porous foam pads, which is very troublesome.

Another relevant vacuum sealing drainage device is known from CN 101390790 A, teaching a suction tube with lateral openings extending in a horizontal direction through a porous foam pad.

Therefore, it is an urgent and important task to improve use of the conventional VSD technology in healing a bleeding wound tissue.

### SUMMARY OF THE INVENTION

It is an objective of the invention to provide a vacuum sealing drainage device for a bleeding wound tissue that is capable of addressing the above-mentioned problems with the conventional vacuum sealing drainage device.

The invention is defined by the independent claim, while preferred embodiments are disclosed by the appended dependent claims.

To achieve the objective of the invention, there is provided a vacuum sealing drainage device for a bleeding wound tissue comprising a porous foam pad configured to contact the bleeding wound tissue, a drainage piping with a side hole, a sealing film, a connector, a duct, a drainage container, a vacuum source, and it further comprises a gel membrane and an outlet pipe. The gel membrane is disposed above the porous foam pad. An opening of the side hole of the drainage piping is contacted with the porous foam pad. The outlet pipe is connected to the drainage piping, and disposed above the gel membrane. The sealing film is disposed on the perimeter of the gel membrane. The outlet pipe is connected to the drainage container via the duct, and the drainage container is connected to the vacuum source via the duct.

In the embodiment of the invention, the drainage piping is disposed between the porous foam pad and the gel membrane.

In a preferred embodiment, the drainage pipe is circular or flat.

In a further embodiment, a flow meter is connected to the duct between the outlet pipe and the drainage container.

In a preferred embodiment, the drainage piping and the gel membrane are integrally formed.

In the invention, a support member is disposed in the porous foam pad, the plurality of support members is hollow and each of said plurality of support members has a plurality of openings disposed on said sidewall. One end of the support member is disposed in the porous foam pad, and the other end of the support member is connected to the drainage piping. Each of said plurality of support members has a sidewall. The plurality of support members is hollow and each of said plurality of support members has a plurality of openings disposed on said sidewall. The plurality of support members extend vertically into the porous foam pad.

In the invention, the drainage piping comprise two pipes integrally formed and connected with each other, each pipe of said drainage piping having a cylindrical sidewall and an axis. The drainage piping is disposed above said porous foam pad, and the axis are oriented parallel to a top surface of said porous foam pad. One end of said outlet pipe is connected to said cylindrical sidewall, the other end of said outlet pipe is connected to the connector, and said connector is connected to said drainage container via said duct. One end of said outlet pipe passes through the gel membrane.

In an embodiment, a bacterial filter and an odor filter are disposed between the drainage container and the vacuum source.

In another embodiment, the drainage piping is #-shaped (number-sign shaped), +-shaped (plus sign-shaped), 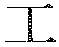 -shaped (H-shaped), or 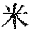 -shaped (star-shaped).

In an embodiment, one to five outlet pipes are connected to the drainage piping, and the outlet pipes and the drainage piping are integrally formed.

In an embodiment, a lower end surface of the support member is a concave contour.

In an embodiment, a sealing cover is disposed on the outlet pipe.

In an embodiment, a sealable sample port is disposed on the outlet pipe.

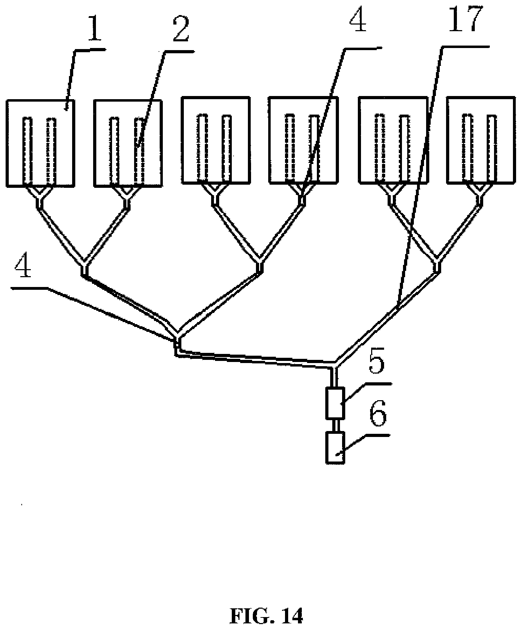

Advantages of the vacuum sealing drainage device for a bleeding wound tissue of the invention are as follow: 1) wound surface is sealed via the gel membrane on the porous foam pad (the gel membrane features very low air permeability, and it can be made by other materials and features very low air permeability), which greatly increases time of maintaining elasticity and a humid environment for the porous foam pad, and is helpful for healing of the wound surface. 2) only surrounding of the gel membrane needs to be sealed with the sealing film (or the gel membrane and surrounding thereof are entirely covered with the sealing film), and the leading-out end of the drainage piping should not be sealed, which reduce possibility of air leakage of the VSD system, and make sealing of the vacuum sealing drainage simple and reliable; moreover, the sealing film has very high permeability, good compatibility with skin, and low sensitization rate, and is helpful for normal metabolism of healthy skin around the wound surface. 3) the outlet pipe is connected to every drainage piping, so there are multiple directions for the outlets, the outlet of each drainage piping can operate as a drainage port, or as a flush port as pipe blockage occurs, which makes drainage and flushing very convenient; meanwhile, a outlet pipe is allocated for flushing during operation, and thus preventing reverse infection as one outlet pipe is used for both drainage and flushing. Side pipes led out from the drainage piping can operate as a sampling channel for drainage objects. 4) the above-mentioned drainage method is more uniform and reasonable in terms of conducting negative pressure and drainage than side drainage method, and thus pipe blockage does not easily occur, especially as the outlet pipe is disposed at intersection of the drainage piping. 5) for mass and irregular wound surface, the outlet pipes are connected to the drainage container via a multi-way connector, which makes drainage very convenient, prevents a problem of a branch-shaped mesh (as shown in FIG. 14), and is convenient for movement of a patient. 6) operation time is reduced, pain of a patient is alleviated, and healing time of the wound surface of the patient is decreased.

### BRIEF DESCRIPTION OF THE DRAWINGS

FIG. 1 is a schematic view of a vacuum sealing drainage device for a bleeding wound tissue of a first embodiment of the invention (a sealing film thereof is not shown);
FIG. 2 illustrates a position relationship among a porous foam pad, a sealing film, and a gel membrane of the invention (in which a dashed part is an invisible part);
FIG 3 illustrates a position relationship among an outlet pipe, a drainage piping, a gel membrane, and a support member of the invention;
FIG. 4 illustrates a position relationship among a outlet pipe, a drainage piping, a gel membrane, a support member, and a porous foam pad of the invention;

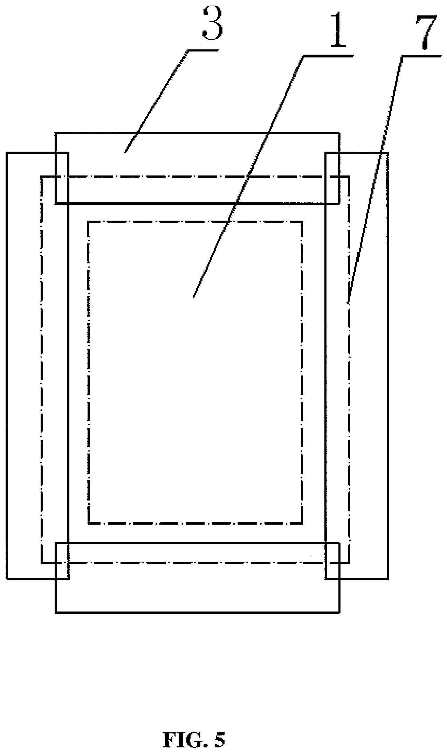

FIG. 5 illustrates another position relationship among a outlet pipe, a drainage piping, a gel membrane, a support member, and a porous foam pad of the invention;
FIG. 6 is a schematic view of a outlet pipe and a sealing cover of a further embodiment;
FIG. 7 is a schematic view of a pair of pipes parallel to each other;
FIG. 8 is a schematic view of a +-shaped drainage piping of another embodiment;
FIG. 9 is a schematic view of a #-shaped drainage piping of another embodiment;
FIG. 10 is a schematic view of a 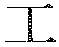 -shaped drainage piping of another embodiment;
FIG. 11 is a schematic view of a 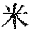 -shaped drainage piping of another embodiment;
FIG. 12 is a schematic view of a vacuum sealing drainage device for a bleeding wound tissue of a further embodiment (a sealing thereof film is not shown);
FIG. 13 illustrates connection relationships of a filter, a drainage container, and a vacuum source; and
FIG. 14 is a schematic view of a branch-shaped mesh formed via a conventional vacuum sealing drainage.

In the drawings, the following reference numbers are used: 1 - porous foam pad; 2 - drainage piping; 3 - sealing film; 4 - connector; 5 - drainage container; 6 - vacuum source; 7 - gel membrane; 8 - outlet pipe; 9 - support member; 10 - burr; 11 - sealing cover; 12 - groove; 13 - opening; 14 - concave contour ; 15 - sampling port; 16 - flow meter; 17 - duct; 18 - bacterial filter; 19 - odor filter; 20 - pressure sensor; 21 - CPU

### DETAILED DESCRIPTION OF THE EMBODIMENTS

Further description of the invention will be given below in conjunction with accompanying drawings, and they are not to be construed as limitation to the invention and are illustrative only. Merits of the invention will be more readily and understandable from the following detailed description.

As shown in the drawings, a vacuum sealing drainage device for a bleeding wound tissue according to an embodiment of the invention includes a porous foam pad 1 configured to contact the bleeding wound tissue, a drainage piping 2 with a side hole, a sealing film 3, a connector 4, a drainage container 5, and a vacuum source 6, and further comprises a gel membrane 7 and an outlet pipe 8. The gel membrane 7 is disposed above the porous foam pad 1. An opening of the side hole of the drainage piping 2 is contacted with the porous foam pad 1. The outlet pipe 8 is connected to the drainage piping 2, and disposed above the gel membrane 7. The sealing film 3 is sealed on the gel membrane 7. The outlet pipe 8 is inserted into the drainage container 5, and the drainage container 5 is connected to the vacuum source 6.

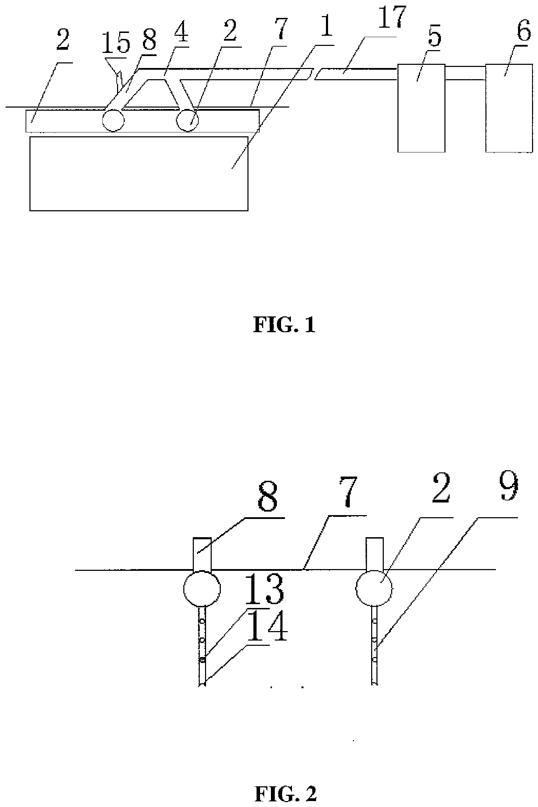

The above-mentioned drainage manner makes drainage extend in all directions so that conducting negative pressure and drainage are more uniform and reasonable than side drainage manner (a conventional drainage device drain or conducts negative pressure at a leading-out end of the drainage piping), so pipe blockage does not easily occurs, especially as the outlet pipe is disposed at intersection of the drainage piping (as shown in FIGS. 1-4).

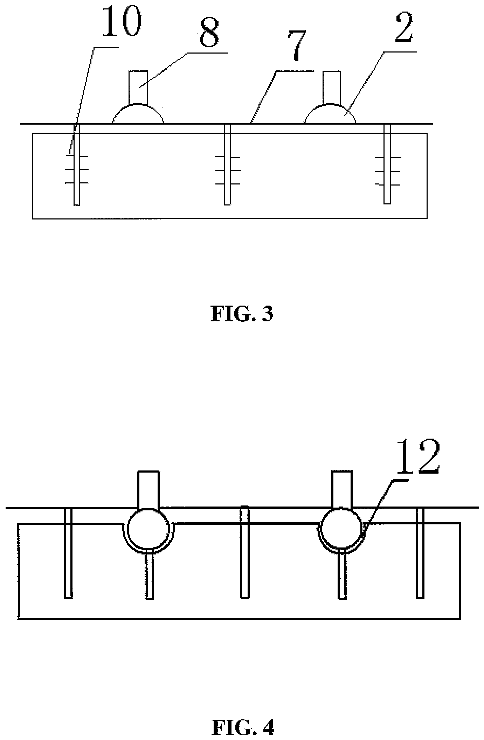

The drainage piping 2 is disposed between the porous foam pad 1 and the gel membrane 7 (as shown in FIG. 4), the drainage piping 2 and the gel membrane 7 are integrally formed (or the drainage piping is not connected to the gel membrane). A groove 12 is disposed in the porous foam pad, and the drainage piping is disposed in the groove in the porous foam pad.

The drainage piping 2 is circular or flat (or employs other structure), and is disposed at the top of the gel membrane 7 and integrally formed with the gel membrane 7 (as shown in FIG 3).

The support member 9 is hollow and is connected to the drainage piping 2. An opening 13 is disposed on the support member 9 (to assist in drainage). A lower end surface of the support member 9 (made of extensible soft materials) is a concave contour (compared with a support with a plane lower end surface, the concave contour increases a contact area with the wound surface and reduces pressure on wound tissues in a vacuum state).

The support member 9 is disposed in the porous foam pad 1. One end of the support member 9 is disposed in the porous foam pad 1, the plurality of support members 9 extend vertically into the porous foam pad 1, and the other end of the support member 9 is fixed on the drainage piping 2 and/or on the gel membrane 7. Multiple burrs 10 are disposed on the support member 9 (as shown in FIGS. 2-4). The structure has the following advantages: firstly, the support member enables the porous foam pad to support between the bleeding wound tissue and the sealing film so that hole gaps and vesicles in the porous foam pad can maintain a normal state or deform slightly, and in turn, liquefaction materials such as exudation, cataclysm, liquefaction necrosis tissue fragments, pus and so on can be conducted into the drainage piping via the vesicles connected with each other whereby reducing possibility of failure of drainage due to pipe blockage. Secondly, the support member enables a elasticity of the porous foam pad for keeping a vacuum environment to be in a normal state, reduces tightly contact between the porous foam pad and the bleeding wound tissue due to negative pressure, prevents unwanted adhesion between the porous foam pad and growing parts of cells of the bleeding wound tissue, is helpful for growth of granulation of the bleeding wound tissue, and decreases healing time of the bleeding wound tissue. Meanwhile, the burr rivets and fixes the drainage piping, the support members, the gel membrane, and the porous foam pad into a whole, and the support member can not be easily pulled out from the porous foam pad.

The sealing film 3 is covered around the gel membrane 7. The method of film posting only needs posting films to surrounding of the gel membrane and is very simple. Using the method, a mesentery method for sealing the drainage piping at the drainage end of the drainage piping is not required in film sealing and posting, which makes sealing more convenient.

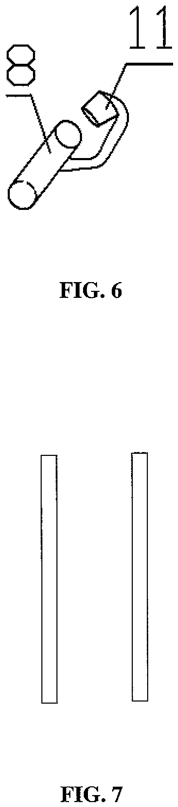

The drainage piping 2 can be one pipe, or formed by two to five pipes disposed parallel to each other. As the drainage piping is one pipe, the structure is applicable to small wound surface. As the drainage piping 2 is formed by two to five pipes disposed parallel to each other (normally two pipes, and three or more drainage pipings for a comparatively large area), the structure is applicable to regular wound surface (as shown in FIG. 7).

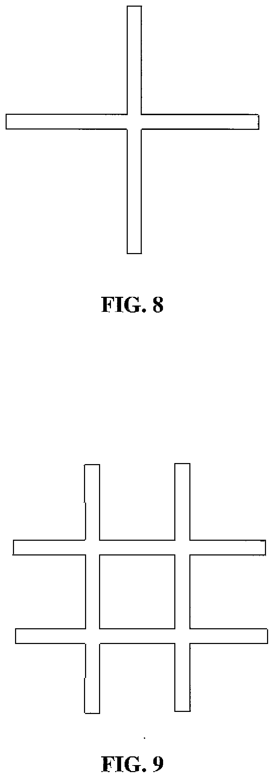

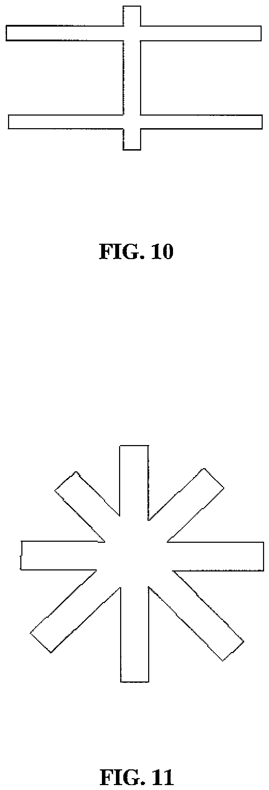

The drainage piping 2 is integrally formed by at least two pipes crossingly connected to each other. For example, the drainage piping 2 is #-shaped, +-shaped, I-shaped, or other intersected structure. The structure makes drainage extend in all directions, and negative pressure conduction more uniform. It is helpful for negative pressure conduction and drainage (as shown in FIGS. 8-11).

One to five outlet pipes 8 are connected to the drainage piping 2, and the outlet pipes 8 and the drainage piping 2 are integrally formed.

A sealing cover 11 is disposed on the outlet pipe 8, and the structure prevents air pollution as the outlet pipe does not drain, or be flushed (as shown in FIG. 6).

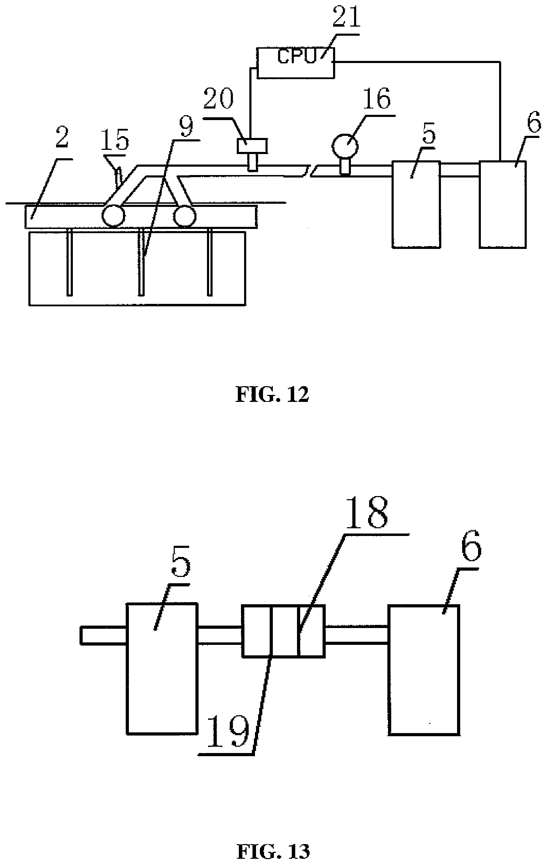

A flow meter 16 is connected to the duct 17 between the outlet pipe 8 and the drainage container 5 (as shown in FIG. 12). Functions of the flow meter are as follow. Firstly, monitor active bleeding of a wound surface whereby preventing hypovolemic shock. Secondly, monitor a mass loss of humor whereby preventing hypoproteinemia caused by a mass loss of albumen. Thirdly, provide data support for fluid and colloid supplement. Fourthly, compared with using a drainage bottom with graduation to record loss of humor, the flow meter operates to record loss of humor within a unit time, and provides timely data support for clinical emergency treatment.

A sealable sampling port 15 is disposed on the outlet pipe.8, which overcomes a problem of the conventional VSD device with no sampling port. During sampling of the conventional VSD device, a connection position of the drainage piping needs to be opened for sampling, which causes a possibility of a new pollution of wound surface. The special sampling port prevents an occurrence of the problem. The sampling port has the following functions: first, performing qualitative analysis on composition of drainage liquid and providing basis for clinical treatment; second, performing drug sensitive test on samples of drainage objects whereby providing an optimum treatment of antibiotics and antibacterial infection (as shown in FIG. 1 and FIG. 12).

A bacterial filter 18 and an odor filter 19 are disposed between the drainage container 5 and the vacuum source 6. The bacterial filter is capable of reducing bacteria, especially anaerobic bacteria, which enter the next drainage channel, such as a central vacuum source, so as to prevent a possibility of pollution of a central vacuum system in a hospital. The odor filter is capable of reducing foul odor generated on the wound surface that overflows from the system (as shown in FIG. 13).

A pressure sensor 20 is connected to the duct 17 has a pressure sensor 20 which is disposed thereon. A CPU 21 is connected to the pressure sensor 20, and the CPU 21 is connected to the vacuum source 6. The pressure sensor controls opening and closing of the vacuum source, or adjusts negative pressure output from the vacuum source according to commands from the CPU by measuring pressure transmitted in the duct from the porous foam pad and processing a pressure signal with the CPU. The negative pressure is transmitted to the porous foam pad via the drainage container and the drainage piping, whereby adjusting a pressure between the vesicle and the hole gap in the porous foam pad.

In use, the support member is inserted into the porous foam pad, the gel membrane and the drainage piping are disposed on the porous foam pad. Surrounding of the gel membrane is sealed via the sealing film. The duct is connected to the outlet pipe on the gel membrane. After the drainage container and the vacuum source are connected, they can be used. During use, a multi-way connector is connected to all of the outlet pipes. If there are four outlet pipes, a five-way connector can be used, and thus they can be connected to the drainage container via the duct. In another manner, only two outlet pipes are used as drainage outlets, and other outlet pipes are used during flushing.

Advantages of the VSD device of the present disclosure is described below via comparison between clinical cases of the VSD device of the present disclosure and those of the conventional VSD device.

The conventional VSD device uses two parallel drainage pipings disposed in the porous foam pad, sealed via the sealing film, and connected to the drainage container and the vacuum source via the connector and the duct.

The VSD device of the present disclosure is as follow. A flat #-shaped drainage piping is disposed above the gel membrane, and the support member is disposed under the gel membrane. The support member has burrs disposed thereon and is inserted into the porous foam pad. The Drainage piping has the outlet pipes disposed thereon, and the sampling port is disposed on the outlet pipe. The outlet pipe is connected to the drainage container and to the vacuum source via the duct. The bacterial filter and the odor filter are disposed between the drainage container and the vacuum source. The multi-way connector is connected the outlet pipes to the drainage container via the duct, and a outlet pipe is left as a flushing port. Surrounding of the gel membrane is sealed by the gel membrane.

There are 180 patients who have soft tissue avulsion injury, open fracture or soft tissue defect and whose courses are 3 months. They are divided into an even group and an odd group according to sequences of treatment. The 90 patients of the odd group are treated with the conventional VSD device (A group), and the 90 patients of the even group are treated with the VSD device of the present disclosure (B group). Observation time is a course of VSD treatment, namely 5 - 7 days. In the above-mentioned course of treatment, comparison is performed in terms of sealing operation time during operation, a pipe blockage rate, a dry rate of the porous foam pad, and percentage of dents on the wound surface. Times of producing one single product are also listed in the following table.

**Table 1: comparison of clinical operation and postoperative effect between the VSD device of the present disclosure and the conventional VSD device**

| Compared item (taking 450 cm ² for example) | Conventional VSD device (Group A) | VSD device of the disclosure (Group B) |
|---|---|---|
| Sealing operation time during operation | 10 ∼ 20 minutes | 3 ∼ 5 minutes |
| Pipe blockage rate | Above 60% | Below 10% |
| Dry rate of porous foam pad | Above 70% | Below 10% |
| percentage of dents on the wound surface | Above 50% | Below 10% |
| Time of producing one single product | 3 ∼ 4 minutes | Within 1 minute |

Compared items are wound surfaces whose areas are approximately 450 cm². Time of producing one single product for the conventional VSD device refers to time of inserting the drainage piping into the porous foam pad, and that for the present disclosure refers to time of combining the gel membrane, the drainage piping, and the outlet pipe with the porous foam pad.

Clinical comparison and analysis of postoperative effects between the VSD device of the disclosure and the conventional VSD device are described as below.

From January 2009 to September 2009, the inventor compares 30 patients having soft tissue defect and concurrent infection respectively treated via the conventional VSD device and the VSD device of the present disclosure. All the patients are treated more than two weeks after their injuries, and this belongs to post processing. An area of the wound surface is larger than 100 cm² and deep into a muscular layer. And all of them have tissue necrosis. The two groups of cases all exclude patients having wound surface on face, hands, feet, and perineum, chronic soft tissue infection, concurrent osteomyelitis, age above 50, and concurrent diabetes. For the conventional VSD device, there are 17 man and 13 woman having ages between 6 and 48, an average age of 27.8, and an average area of wound surface of 182.3 cm². For the VSD device of the present disclosure, there are 16 man and 14 woman having ages between 7 and 49, an average age of 28.3, and an average area of wound surface of 169.8 cm². Physical conditions and wound surface of the two groups of cases are approximately the same, and thus having comparability. T-test is used for statistical treatment.

Tables 2-1 and 2-2 illustrate injury conditions, injury parts, and repairing methods of the two groups of cases. Table 2-3 lists cleaning time of wound surfaces of the two groups. The cleaning time of wound surface refers to time from treatment to closing of wound surface of soft tissue defect and infection. Standards of cleaning of the wound surface and closing thereof are as follow. The wound surface is flat and clean, and with no necrotic tissues or exudates occurs; edema of the wound surface disappears; granulation tissue of the wound surface is fresh, has pink granules, and easily bleeds once being contacted; the wound surface ensures successful secondary suture, skin grafting, and skin flap transfer. The time is calculated in days from a day finding wound surface infection or debridement, and removing necrotic tissue, to one day before a day repairing wound (secondary suture, skin grafting, and skin flap transfer).

**Table 2-1 Injury reasons and complications**

| injury reasons | | | | complications | | |
|---|---|---|---|---|---|---|
| Group | Traffic accident | Machine | Pressed by a heavy object | Bone exposure | Fracture | multiple trauma |
| Conventional VSD group | 18 | 10 | 2 | 21 | 8 | 19 |
| VSD group of the disclosure | 15 | 11 | 4 | 19 | 10 | 20 |

**Table 2-2 Wound parts and repairing methods**

| Group | Wound parts | | Repairing methods of wound surface | | |
|---|---|---|---|---|---|
| | Limb | Body | secondary suture + skin grafting | skin grafting | Skin flap transfer |
| Conventional VSD group | 26 | 4 | 5 | 22 | 3 |
| VSD group of the disclosure | 25 | 5 | 4 | 25 | 1 |

**Table 2-3 Distribution of cleaning time of wound surfaces**

| Time (day) | 0-∼6 | 7∼13 | 14∼20 | 21∼27 | 28∼34 | 35∼41 | Sum |
|---|---|---|---|---|---|---|---|
| Conventional VSD group | 8 | 14 | 4 | 3 | 1 | 0 | 30 |
| VSD group of the disclosure | 13 | 10 | 5 | 2 | 0 | 0 | 30 |

Cleaning time of the wound surfaces of the conventional VSD group are 5 - 15 days. The wound surfaces are reduced at different degrees, 22 patients undergo skin grafting, and 3 patients undergo skin flap transfer. Cleaning time of the wound surfaces of the VSD group of the present disclosure are 3-10 days, which is significantly decreased in comparison with the conventional VSD group (P<0.01, the difference between the two groups has statistical significance). Decreases of the wound surfaces of the VSD group of the present disclosure are more obvious, 25 patients undergo skin grafting, and 1 patient undergoes skin flap transfer. The above results indicate that treatment effects of using the VSD device of the present disclosure to handle soft tissue defect with concurrent infection is better than that of the conventional VSD device.

It can be known from Table 1, Table 2-1, Table 2-2, and Table 2-3 that the VSD device of the present disclosure has a qualitative leap in terms of ease of clinical operation, understanding and application of principles, reasonable arrangement of its structure and so on, in comparison with domestic and oversea products and various related technical documents and bibliographies. Merits or advantages are beyond imagination of those skilled in the art.

## Claims

1. A vacuum sealing drainage device for a bleeding wound tissue, the device comprising: a porous foam pad (**1**) configured to contact with said the bleeding wound tissue, a drainage piping (**2**), a sealing film (**3**), a connector (**4**), a duct (**17**), a drainage container (**5**), a vacuum source (**6**), a gel membrane (7), at least one outlet pipe (8), and a plurality of support members (9), each of said plurality of support members (9) having a sidewall;
wherein
said gel membrane (**7**) is disposed above said porous foam pad (**1**);
said drainage piping (2) comprise two pipes integrally formed and connected with each other, each pipe of said drainage piping (2) having a cylindrical sidewall and an axis;
said drainage piping (2) is disposed above said porous foam pad (1), and the axis are oriented parallel to a top surface of said porous foam pad (1);
said plurality of support members (9) is hollow and each of said plurality of support members (9) has a plurality of openings (13) disposed on said sidewall;
one end of each of said plurality of support members (9) is disposed in said porous foam pad (1), and said plurality of support members (9) extends vertically with said plurality of openings (13) into the porous foam pad (1);
the other end of each of said plurality of support members (9) is connected to said drainage piping (2);
one end of said outlet pipe (**8**) is connected to said cylindrical sidewall;
said sealing film (**3**) is disposed on the perimeter of said gel membrane (7);
the other end of said outlet pipe (**8**) is connected to said connector (4), and said connector (4) is connected to said drainage container (**5**) via said duct (**17**);
said drainage piping (2) is disposed between said porous foam pad (1) and said gel membrane (7); and
said one end of said outlet pipe (8) passes through said gel membrane (7); and
said drainage container (**5**) is connected to said vacuum source (**6**) via said duct (**17**).

2. The vacuum sealing drainage device of claim 1, wherein a flow meter (**16**) is connected to said duct (**17**) between said outlet pipe (**8**) and said drainage container (**5**).

3. The vacuum sealing drainage device of claim 1, wherein said drainage piping (**2**) and said gel membrane (**7**) are integrally formed.

4. The vacuum sealing drainage device of claim 1, wherein said drainage piping (**2**) comprises two to five pipes disposed parallel to each other.

5. The vacuum sealing drainage device of claim 1, wherein a bacterial filter (**18**) and an odor filter (**19**) are disposed between said drainage container (**5**) and said vacuum source (**6**).

6. The vacuum sealing drainage device of claim 1, wherein one to five outlet pipes (**8**) are connected to said drainage piping (**2**), and said one to five outlet pipes (**8**) and said drainage piping (**2**) are integrally formed.

7. The vacuum sealing drainage device of claim 1, wherein a lower end surface of each of said plurality of support members (**9**) has a concave contour (**14**).

8. The vacuum sealing drainage device of claim 1, wherein a sealable sample port (15) is disposed on said outlet pipe (**8**).

## Patentansprüche

1. Vakuumversiegelnde Drainagevorrichtung für ein blutendes Wundgewebe, wobei die Vorrichtung umfasst:
ein poröses Schaumstoffkissen (1), das konfiguriert ist, um in Kontakt mit dem blutenden Wundgewebe zu sein, ein Drainageleitungssystem (2), eine Dichtfolie (3), ein Verbindungsstück (4), eine Rohrleitung (17), ein Drainagebehälter (5), eine Vakuumquelle (6), eine Gelmembran (7), mindestens ein Ablaufrohr (8) und eine Vielzahl von Trägerelementen (9), wobei jedes der Vielzahl von Trägerelementen (9) eine Seitenwand aufweist;
wobei
die Gelmembran (7) über dem porösen Schaumstoffkissen (1) angeordnet ist;
das Drainageleitungssystem (2) zwei Rohre umfasst, die in einem Stück gebildet und miteinander verbunden sind, wobei jedes Rohr des Drainageleitungssystems (2) eine zylindrische Seitenwand und eine Achse aufweist;
das Drainageleitungssystem (2) über dem porösen Schaumstoffkissen (1) angeordnet ist, und die Achse parallel zu einer oberen Fläche des porösen Schaumstoffkissens (1) ausgerichtet ist;
die Vielzahl von Trägerelementen (9) hohl ist und jedes der Vielzahl von Trägerelementen (9) eine Vielzahl von Öffnungen (13) aufweist, die auf der Seitenwand angeordnet sind;
ein Ende jedes der Vielzahl von Trägerelementen (9) in dem porösen Schaumstoffkissen (1) angeordnet ist, und die Vielzahl von Trägerelementen (9) sich vertikal mit der Vielzahl der Öffnungen (13) in das poröse Schaumstoffkissen (1) erstreckt;
das andere Ende der Vielzahl von Trägerelementen (9) mit dem Drainageleitungssystem (2) verbunden ist;
ein Ende des Ablaufrohrs (8) mit der zylindrischen Seitenwand verbunden ist;
die Dichtfolie (3) auf dem Umfang der Gelmembran (7) angeordnet ist;
das andere Ende des Ablaufrohrs (8) mit dem Verbindungsstück (4) verbunden ist, und das Verbindungsstück (4) mit dem Drainagebehälter (5) über die Rohrleitung (17) verbunden ist;
das Drainageleitungssystem (2) zwischen dem porösen Schaumstoffkissen (1) und der Gelmembran (7) angeordnet ist; und
das eine Ende des Ablaufrohrs (8) durch die Gelmembran (7) verläuft; und
der Drainagebehälter (5) mit der Vakuumquelle (6) über die Rohrleitung (17) verbunden ist.

2. Vakuumversiegelnde Drainagevorrichtung nach Anspruch 1, wobei ein Durchflussmesser (16) an der Rohrleitung (17) zwischen dem Ablaufrohr (8) und dem Drainagebehälter (5) angeschlossen ist.

3. Vakuumversiegelnde Drainagevorrichtung nach Anspruch 1, wobei das Drainageleitungssystem (2) und die Gelmembran (7) als ein Stück gebildet sind.

4. Vakuumversiegelnde Drainagevorrichtung nach Anspruch 1, wobei das Drainageleitungssystem (2) zwei bis fünf Rohre umfasst, die parallel zueinander angeordnet sind.

5. Vakuumversiegelnde Drainagevorrichtung nach Anspruch 1, wobei ein Bakterienfilter (18) und ein Geruchsfilter (19) zwischen dem Drainagebehälter (5) und der Vakuumquelle (6) angeordnet sind.

6. Vakuumversiegelnde Drainagevorrichtung nach Anspruch 1, wobei ein bis fünf Ablaufrohre (8) an das Drainageleitungssystem (2) angeschlossen sind, und die ein bis fünf Ablaufrohre (8) und das Drainageleitungssystem (2) in einem Stück gebildet sind.

7. Vakuumversiegelnde Drainagevorrichtung nach Anspruch 1, wobei eine untere Endfläche jedes der Vielzahl von Trägerelementen (9) eine konkave Kontur (14) aufweist.

8. Vakuumversiegelnde Drainagevorrichtung nach Anspruch 1, wobei ein abdichtbarer Probenentnahmeanschluss (15) an dem Ablaufrohr (8) angeordnet ist.

## Revendications

1. Dispositif de drainage à étanchéité à vide pour tissu de blessure saignante, le dispositif comprenant : un coussinet de mousse poreuse (1) configuré pour entrer en contact avec ledit tissu de blessure saignante, un tuyautage de drainage (2), un film d'étanchéité (3), un connecteur (4), une conduite (17), un récipient de drainage (5), une source de vide (6), une membrane de gel (7), au moins un tuyau de sortie (8), et une pluralité d'éléments de support (9), chacun de ladite pluralité d'éléments de supports (9) ayant une paroi latérale ;
où
ladite membrane de gel (7) est disposée au-dessus du coussinet de mousse poreuse (1) ;
ladite tuyauterie de drainage (2) comprend deux tuyaux formés de manière intégrée et connectés l'un avec l'autre, chaque tuyau dudit tuyautage de drainage (2) ayant une paroi latérale cylindrique et un axe ;
ledit tuyautage de drainage (2) est disposé au-dessus dudit coussinet de mousse poreuse (1), et l'axe sont orientés parallèlement à une surface de la partie supérieure dudit coussinet de mousse poreuse (1) ; ladite pluralité d'élément de support (9) est creuse et chacun de ladite pluralité d'éléments de support (9) a une pluralité d'ouvertures (13) disposées sur ladite paroi latérale ;
une extrémité de chacun de ladite pluralité d'éléments de support (9) est disposée dans ledit coussinet de mousse poreuse (1), et ladite pluralité d'éléments de support (9) s'étendent verticalement avec ladite pluralité d'ouvertures (13) dans le coussinet de mousse poreuse (1) ;
l'autre extrémité de chacun de ladite pluralité d'éléments de support (9) est connectée à ladite tuyauterie de drainage (2) ;
une extrémité dudit tuyau de sortie (8) est connectée à ladite paroi latérale cylindrique ;
ledit film d'étanchéité (3) est disposé sur le périmètre de la dite membrane de gel (7) ;
l'autre extrémité dudit tuyau de sortie (8) est connectée audit connecteur (4), et ledit connecteur (4) est connecté audit récipient de drainage (5) via ladite conduite (17) ;
ladite tuyauterie de drainage (2) est disposée entre ledit coussinet de mousse poreuse (1) et ladite membrane de gel (7) ; et
ladite une extrémité dudit tuyau de sortie (8) passe à travers ladite membrane de gel (7) ; et
ledit récipient de drainage (5) est connecté à ladite source de vide (6) via ladite conduite (17).

2. Dispositif de drainage à étanchéité à vide selon la revendication 1, dans lequel un débitmètre (16) est connecté à ladite conduite (17) entre ledit tuyau de sortie (8) et ledit récipient de drainage (5).

3. Dispositif de drainage à étanchéité à vide selon la revendication 1, dans lequel ladite tuyauterie de drainage (2) et ladite membrane de gel (7) sont formées de manière intégrée.

4. Dispositif de drainage à étanchéité à vide selon la revendication 1, dans lequel ladite tuyauterie de drainage (2) comprend deux à cinq tuyaux disposés parallèlement l'un à l'autre.

5. Dispositif de drainage à étanchéité à vide selon la revendication 1, dans lequel un filtre bactérien (18) et un filtre d'odeurs (19) sont disposés entre ledit récipient de drainage (5) et ladite source de vide (6).

6. Dispositif de drainage à étanchéité à vide selon la revendication 1, dans lequel un à cinq tuyaux de sortie (8) sont connectés à ladite tuyauterie de drainage (2), et lesdits un à cinq tuyaux de sortie (8) et ladite tuyauterie de drainage (2) sont formés de manière intégrée.

7. Dispositif de drainage à étanchéité à vide selon la revendication 1, dans lequel une surface d'extrémité inférieure de chacun de ladite pluralité d'éléments de support (9) a un contour concave (14).

8. Dispositif de drainage à étanchéité à vide selon la revendication 1, dans lequel une porte d'échantillon scellable (15) est disposée sur ledit tuyau de sortie (8).
